# EUROPEAN PATENT SPECIFICATION

(11) **EP 3 145 554 B1**
(45) Date of publication and mention of the grant of the patent: **10.07.2019**
(21) Application number: 15796802.5
(22) Date of filing: 22.05.2015
(51) Int. Cl.: A61L 2/26, A61B 90/70, A61B 1/12

(54) **DECONTAMINATION SYSTEM CONNECTORS**
VERBINDER FÜR DEKONTAMINATIONSSYSTEM
RACCORDS DE SYSTÈME DE DÉCONTAMINATION

(30) Priority: 23.05.2014 US 201462002677 P
(43) Date of publication of application: 29.03.2017
(73) Proprietor: Medivators Inc., Minneapolis, MN 55447 (US)
(72) Inventor: DEPREY, Eric John, Prior Lake, Minnesota 55372 (US); HARM, William H., Robbinsdale, Minnesota 55422 (US)
(74) Representative: Liesegang, Eva
(86) International application number: PCT/US2015/032276
(87) International publication number: WO 2015/179824

(56) References cited:
- US-A- 5 552 115
- US-A1- 2003 026 729
- US-A1- 2009 107 529
- US-A1- 2009 119 856
- US-A1- 2011 135 537
- US-B1- 6 485 684
- US-B1- 6 485 684
- US-B2- 7 229 591

## Description

### TECHNICAL FIELD

The present invention relates to decontamination of medical devices. More particularly, the present invention relates to containers and connectors associated with the decontamination of medical devices.

### BACKGROUND

Robust medical instruments are often sterilized at high temperatures. Commonly, the instruments are sterilized in a steam autoclave under a combination of high temperature and pressure. While such sterilization methods are very effective for more durable medical instruments, advanced medical instruments formed of rubber and plastic components with adhesives are delicate and wholly unsuited to the high temperatures and pressures associated with a conventional steam autoclave. Steam autoclaves have also been modified to operate under low pressure cycling programs to increase the rate of steam penetration into the medical devices or associated packages of medical devices undergoing sterilization. Steam sterilization using gravity, high pressure or pre-vacuum create an environment where rapid changes in temperature can take place. In particular, highly complex instruments which are often formed and assembled with very precise dimensions, close assembly tolerances, and sensitive optical components, such as endoscopes, may be destroyed or have their useful lives severely curtailed by harsh sterilization methods employing high temperatures and high or low pressures.

Further, endoscopes can also present problems in that such devices typically have numerous exterior crevices and interior lumens which can harbor microbes. Microbes can be found on surfaces in such crevices and interior lumens as well as on exterior surfaces of the endoscope. Other medical or dental instruments which comprise lumens, crevices, and the like can also provide challenges for decontaminating various internal and external surfaces that can harbor microbes.

US 2009/0119856 A1 discloses an apparatus for disinfecting an endoscope comprising a washing bath, in which the endoscope can be mounted, a nozzle unit for connecting to a port of the endoscope and an automatic brush mechanism for mechanical cleaning of a lumen of the endoscope by a cleaning brush introduced through the nozzle unit.

US 6485684 B1 describes a fluid connection system for disinfecting a lumened article like an endoscope. The fluid connection system comprises a chamber for receiving the endoscope, which contains a plurality of fluid outlets for disinfecting exterior surfaces of the endoscope as well as a plurality of fluid ports for disinfecting interior lumens of the endoscope.

US 2003/0026729 A1 describes a reservoir for a decontaminating substance that can be connected to a lumen via an adaptor piece with a truncated cone. The truncated cone forms a hole, into which the lumen fits. The surface of the cone can be partially textured, can be made of a permeable material and its width can be reduced in certain parts to increase the permeability.

In US 005552115 A, a decontamination system comprising tubes with fittings for a connection to an item to be sterilized is disclosed, wherein the fittings include a sleeve made from a porous material. The sleeve is designed to form a firm connection with an annular surface of a port of the item, which is secured by friction.

### SUMMARY

The present invention provides a lumen device decontamination system according to claim 1 and a decontamination method according to claim 14. A lumen device decontamination system includes a lumen device container defining a lumen device receiving area and a fluid connector connected to the lumen device container so as to deliver a decontaminating fluid to the lumen device receiving area. The fluid connector includes a fluid passage suitable for conducting flow of the decontaminating fluid and a means for connecting the fluid connector to a lumen port of a medical device positioned in the lumen device receiving area.

In one embodiment, a lumen device decontamination system for decontaminating a medical device having one or more lumens extending there-through includes a lumen device container including a lumen device receiving area, a fluid passage, and a biased actuated connector in fluid communication with the fluid passage. The biased actuated connector is configured to fluidly connect the fluid passage to the medical device in the lumen device receiving area. The bias actuated connector is movable between a first and second position and including a biasing element to bias the biased actuated connector in the first position. The biased actuated connector extends towards the lumen device receiving area in the first position and retracts from the lumen device receiving area in the second position.

In another embodiment, a method for decontaminating a medical device includes positioning a medical device in a receiving area of a lumen device container, retracting a first biased actuated connector having a first connector end, aligning a first lumen port of the medical device with the first connector end, and releasing the retracted first biased actuated connector to engage with the first lumen port.

In another embodiment, a lumen device decontamination system for decontaminating a medical device having one or more lumens extending there-through includes a lumen device container defining a lumen device receiving area and a retractable connector movable between first and second positions. The retractable connector includes a fluid passage suitable for delivering a decontaminating fluid; and a connector end positioned at an end of the fluid passage. An actuator is operably connected to the retractable connector to actuate the retractable connector between the first and second positions. The retractable connector is closer to the lumen device receiving area in the first position as compared to the second position.

In another embodiment, a method includes positioning a medical device having a first lumen extending there-through in a receiving area of a container, aligning a first lumen port of the medical device with a first connector end of a first retractable connector in a first retracted position; and extending the first retractable connector to engage with the first lumen port, wherein the first lumen port is in fluid communication with the first lumen.

In another embodiment, a lumen device decontamination system for decontaminating a medical device having one or more lumens extending there-through includes a lumen device container including a lumen device receiving area configured to receive a medical device; and a fluid connector connected to the lumen device container and configured to deliver a decontaminating fluid to the lumen device receiving area. The fluid connector includes a fluid passage suitable for conducting flow of the decontaminating fluid; and a connector end positioned at an end of the fluid passage, wherein the connector end is configured to fluidly connect the medical device and the fluid passage, and wherein less than 100% of the decontamination fluid flow through the fluid passage flows through the one or more lumens of the medical device.

In another embodiment, a method includes positioning a medical device having one or more lumens extending there-through in a lumen device receiving area of a lumen device container; and positioning a connector end of a fluid connector proximate a lumen port of the medical device such that the connector end is held loosely with respect to the lumen port so as to allow fluid flow from the fluid conductor to flow both through the lumen port and around the lumen port.

In another embodiment, a lumen device decontamination system for decontaminating a medical device having one or more lumens extending there-through includes a lumen device container defining a lumen device receiving area, and a fluid connector connected to the lumen device container so as to deliver a decontaminating fluid to the lumen device receiving area. The fluid connector includes a fluid passage suitable for conducting flow of the decontaminating fluid, and a connector end positioned at an end of the fluid passage. The connector end includes a textured contact surface operable for contacting a lumen port of a medical device positioned in the lumen device receiving area.

In another embodiment, a method includes positioning a medical device having one or more lumens extending there-through in a lumen device receiving area of a lumen device container; and positioning a connector end of a fluid connector proximate a lumen port of the medical device such that a textured contact surface of the connector end contacts the lumen port of the medical device so as to allow fluid flow from the fluid conductor to flow both through the lumen port and around the lumen port.

In another embodiment, a lumen device decontamination system for decontaminating a medical device having one or more lumens extending there-through includes a lumen device container defining a lumen device receiving area and a fluid connector connected to the lumen device container so as to deliver a decontaminating fluid to the lumen device receiving area. The fluid connector includes a fluid passage suitable for conducting flow of the decontaminating fluid and a connector end positioned at an end of the fluid passage. The connector end comprises a concave contact surface operable for contacting a lumen port of a medical device positioned in the lumen device receiving area.

In another embodiment, a method includes positioning a medical device having one or more lumens extending there-through in a lumen device receiving area of a lumen device container, and positioning a connector end of a fluid connector proximate a lumen port of the medical device such that a concave contact surface of the connector end contacts the lumen port of the medical device so as to allow fluid flow from the fluid conductor to flow through the lumen port.

While multiple embodiments are disclosed, still other embodiments of the present invention will become apparent to those skilled in the art from the following detailed description, which shows and describes illustrative embodiments of the invention. Accordingly, the drawings and detailed description are to be regarded as illustrative in nature and not restrictive.

### BRIEF DESCRIPTION OF THE DRAWINGS

FIG. 1 is a schematic view of a system for decontaminating a medical device.
FIG. 2 is a perspective view of a container for use in the system of FIG. 1.
FIG. 3 is a perspective view of an embodiment of a cradle for use in the container of FIG. 2.
FIG. 4A is a perspective view of the cradle of FIG. 3 in an open position.
FIG. 4B is a perspective view of the cradle of FIG. 3 in a closed position.
FIG. 5 is a perspective view of another embodiment of a cradle for use on the container of FIG. 2.
FIG. 6 is an enlarged perspective view of a portion of the cradle of FIG. 5.
FIG. 7A is a perspective view of an embodiment of a lumen device and a fluid connector.
FIG. 7B is a cross-sectional view of another embodiment of a lumen device and a fluid connector.
FIG. 8 is an enlarged perspective view of another embodiment of a lumen device and a fluid connector.
FIG. 9A is an enlarged perspective view of another embodiment of a lumen device and a fluid connector with the fluid connector disconnected from the lumen device.
FIG. 9B is an enlarged perspective view of the lumen device and the fluid connector of FIG. 9A, with the fluid connector connected to the lumen device.
FIGS. 10, 11 and 12 are cross-sectional views of another embodiment of a fluid connector.

### DETAILED DESCRIPTION

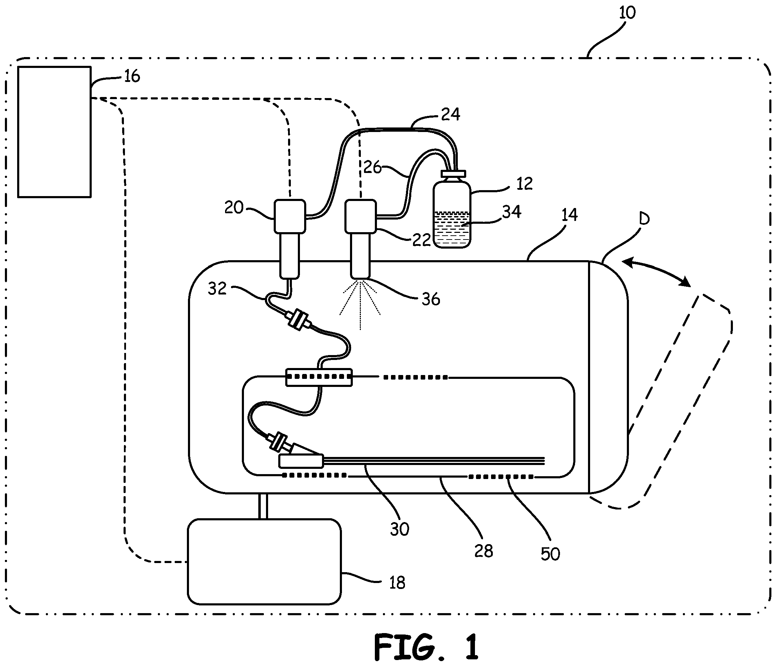

FIG. 1 is a schematic view of one embodiment of a system 10 for decontaminating a medical, dental, or other device having one or more lumens extending there-through. The system includes a reservoir 12, a decontamination chamber 14, a system control system 16, an environmental monitoring and control system 18, and vaporizers 20 and 22 which are connected to reservoir 12 by conduits 24 and 26. A container 28 containing a lumen device 30 for decontamination may be placed within the decontamination chamber 14. In the illustrated embodiment, the container 28 can include a plurality of openings or pores 50. The reservoir 12 may be in fluid communication with the decontamination chamber 14 via vaporizer 22. The reservoir 12 may also be in fluid communication with one or more lumens extending through the lumen device 30 via vaporizer 20 and fluid conduit 32.

The system control system 16 provides control signals to and/or receives condition sensing and equipment status signals from the reservoir 12, decontamination chamber 14, environmental monitoring and control system 18, vaporizer 20 and vaporizer 22. In some embodiments, the system 10 can be assembled in a device small enough to sit on a tabletop or counter. For example, the decontamination chamber 14 may have an interior volume of less than about ten cubic feet. The lumen device 30 to be decontaminated can be placed into the decontamination chamber 14 by opening the door D and placing the lumen device 30 on a rack or other supporting assembly in the interior of the decontamination chamber 14. In some embodiments, lumen device 30 may be enclosed in container 28 before being placed in the decontamination chamber 14.

The chemical reservoir 12 may be a holding tank or other assembly configured to hold a decontaminating substance 34. In some embodiments, the decontaminating substance 34 can be a chemical or other substance suitable for use in a sterilization process that complies with the International Organization for Standardization (ISO) standard ISO/TC 198, Sterilization of Healthcare Products and/or the Association for the Advancement of Medical Instrumentation (AAMI) standard ANSI/AAMI/ISO 11140-1:2005, "Sterilization of Healthcare Products - Chemical Indicators - Part I: General Requirements" (Arlington, VA: AAMI 2005). In some embodiments, the decontaminating substance 34 can be a room temperature (e.g., 20°C to 25°C) substance that can be dispersed as a fluid, such as a liquid, a vapor, or a combination thereof (such as a fog) during the decontamination process. Suitable substances for the decontaminating substance 34 include hydrogen peroxide (H₂O₂) and peracetic acid (PAA).

The system control system 16 controls delivery of the decontaminating substance 34 from the reservoir 12 to vaporizer 22. The decontaminating substance 34 may be pushed or pulled into vaporizer 22.

System control system 16 can also control delivery of the decontaminating substance 34 from the reservoir 12 to vaporizer 20. Similar to vaporizer 22, the decontaminating substance 34 may be pushed or pulled into vaporizer 20. Vaporizer 20 is in fluid communication with at least one lumen of lumen device 30. As described herein, during a decontamination process, the decontaminating substance 34 flows from vaporizer 20 into one or more lumens of the lumen device 30 to decontaminate the lumens.

In some embodiments, the decontaminating substance 34 can flow concurrently from reservoir 12 to vaporizers 20 and 22 and subsequently to decontamination chamber 12 and lumen device 30. In other embodiments, the flow of the decontaminating substance 34 to vaporizer 20 may initiate before or after the initiation of flow of the decontaminating substance 34 to vaporizer 22. The decontaminating substance from vaporizer 20 may decontaminate the interior or lumen surfaces of the lumen device 30 and the decontaminating substance from vaporizer 22 may decontaminate the exterior surfaces of lumen device 30 as well as the surfaces of packaging 28. The amount of decontamination substance 34 introduced into the decontamination chamber 14, the lumen device 30 or a combination thereof can be controlled by the system control system 16 by controlling the amount of the decontamination substance 34 fed or delivered to vaporizers 20 and 22. The rate and amount of decontaminating substance 34 delivered to vaporizers 20 and 22 may be preprogrammed into the system control system 16 or may be manually entered into the system control system 16 by a user of the system 10.

To decontaminate a lumen device, such as a medical, dental or other device, the lumen device 30 may be sealed within the container 28 and placed in the decontamination chamber 14. The lumen device 30 is then subjected to a decontamination process which may include one or more decontamination cycles. A suitable cycle may include adjusting the pressure of the decontamination chamber 14 to a suitable range, such as to a pressure less than 10 Torr, conditioning using plasma, and introducing the decontaminating substance 34 into the decontamination chamber 14 via vaporizer 22 and nozzle 36 and introducing the decontaminating substance 34 into the lumen device 30 via vaporizer 20 and conduit 32. The decontamination substance 34 may be held within the decontamination chamber 14 for a period of time to facilitate the decontamination of the lumen device 30, and in particular, the exterior surfaces of the lumen device 30. Similarly, the decontamination substance 34 may be held within the lumen device 30 for a period of time to facilitate the decontamination of the interior surfaces or lumen(s) of the lumen device 30. When the decontaminating substance 34 has been held in the decontamination chamber 14 for the desired or programmed amount of time, the system control system 16 can vent the decontamination chamber 14 to a higher, but sub-atmospheric pressure. The system controller 16 can then hold the pressure within the decontamination chamber 14 for a period of time to further facilitate the decontamination of the load. Following the hold period, the system control system 16 may evacuate the decontamination chamber 14 to remove the decontamination substance residuals from the decontamination chamber 14 which may also include a plasma treatment to further enhance the removal of the substance residuals, followed by venting the decontamination chamber 14. This cycle or steps within may be repeated or extended as part of a comprehensive cycle.

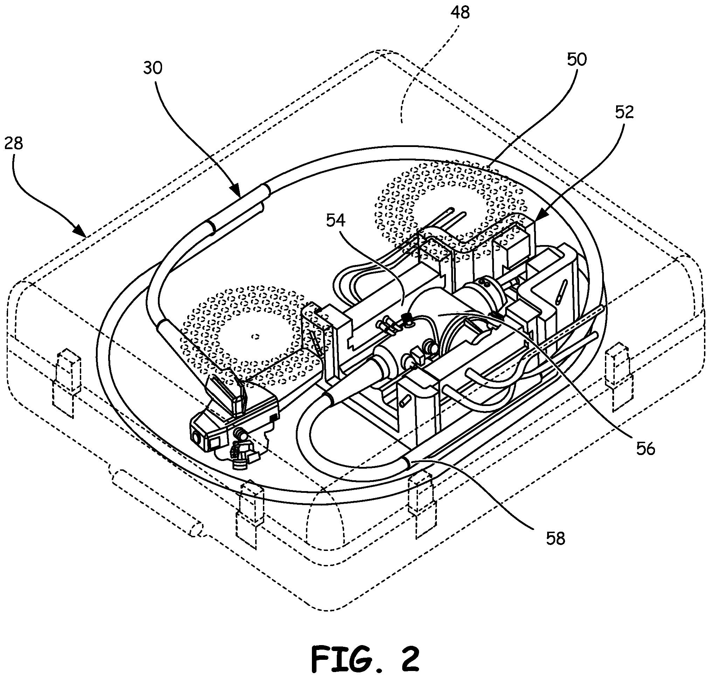

FIG. 2 is a perspective view of the container 28 containing the lumen device 30. The container 28 is sized so that the lumen device 30 to be decontaminated fits within the container 28. Container 28 may be generally described as having a top, a bottom, and four sides extending between the top and bottom to create a cube structure. However, container 28 may have any suitable shape which encloses the lumen device 30. In some embodiments, container 28 may be formed from a rigid material such that container 28 has rigid or structured shape. Alternatively, container 28 may be formed from a flexible material such that container 28 has a flexible shape. In some embodiments, the container 28 may be a terminal package. Suitable materials for container 28 include but are not limited to a polymeric non-woven sheet, such as spun-bonded polyethylene (e.g., Tyvek®, sold by E.I. du Pont de Nemours and Company, Wilmington, Del.), and polymeric materials such as polyester and polypropylene. Suitable materials for container 28 having a rigid or structured shape include but are not limited to various metals such as aluminum and stainless steel and various polymers in rigid form such as polyethylene and polypropylene.

The lumen device 30 may be positioned within the container 28 and subjected to one or more decontamination cycles. Suitable lumen devices 30 include any medical, dental or other device having at least one lumen extending through at least a portion of the device. In some embodiments, the lumen device 30 may include at least one lumen extending the entire length of the device. For example, the lumen device 30 may be an endoscope.

Container 28 may be configured to prevent or reduce microbes and/or other contaminants from entering the container 28. In some embodiments, the container 28 can include a material suitable for allowing flow of a decontaminating substance, such as hydrogen peroxide (H₂O₂) and/or peracetic acid (PAA), into the interior 48 of container 28 and blocking or reducing the flow of contaminants into interior 48. In the illustrated embodiment, the container 28 includes a plurality of openings or pores 50 for allowing flow of the decontaminating substance 34 (FIG. 1) into the container 28. In some embodiments, the pores 50 may be sized so as to allow the decontaminating substance 34 and/or air to communicate into and out of container 28 as well as prevent microbes from entering container 28.

In some embodiments, the container 28 includes a cradle 52 positioned within the interior 48 of the container 28. The cradle 52 defines a lumen device receiving area 54 for receiving the lumen device 30. The lumen device 30 can include a head unit 56 and one or more fluid conduits 58 extending from and fluidly connected to the head unit 56. In the illustrated embodiment, the head unit 56 of the lumen device 30 is positioned in the lumen device receiving area 54 of the cradle 52, and the fluid conduit 58 is positioned outside of the lumen device receiving area 54 yet still inside the interior 48 of the container 28. In alternative embodiments, the lumen device 30 can be replaced by different lumen devices having different constructions. The cradle 52 can be modified to accommodate the different lumen devices. In some embodiments, the container 28 and the cradle 52 can be reusable for multiple decontamination processes. In other embodiments, the container 28 and the cradle 52 can be single use. That is, in other embodiments, the container 28 and the cradle 52 can be disposed of following a first or single use.

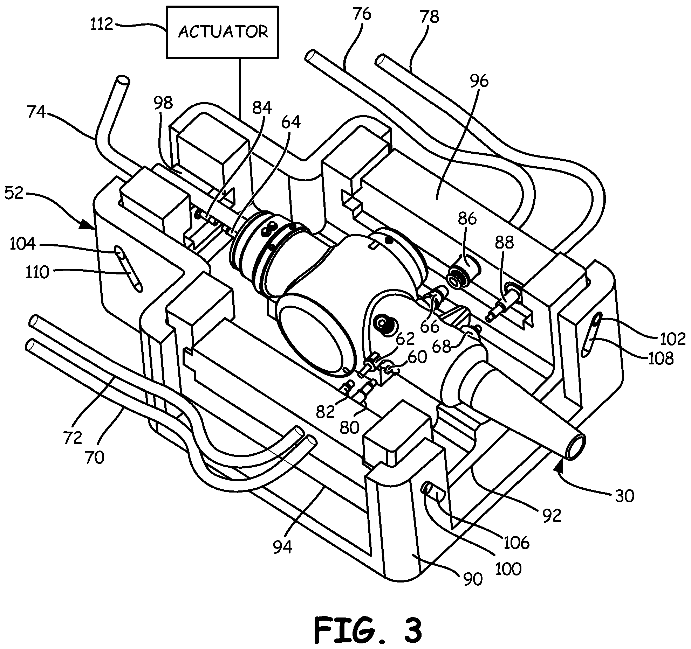

FIG. 3 is a perspective view of an embodiment of the cradle 52 with the container 28 (shown in FIG. 2) removed for clarity. The lumen device 30 may include one or more ports, such as ports 60, 62, 64, 66, 68. The ports 60, 62, 64, 66, 68 are lumen ports for allowing flow through lumens extending through the lumen device 30. The ports 60, 62, 64, 66, 68 can have a variety of positions, shapes, and functions. In some embodiments, the port 60 may be an air/water channel port, the port 62 may be a water channel port, the port 64 may be an air channel port, the port 66 may be a suction channel port, and the port 68 may be an auxiliary/forward water channel port.

The cradle 52 includes fluid passages or conduits 70, 72, 74, 76, 78, each having a connector end 80, 82, 84, 86, 88, respectively. The fluid passages 70, 72, 74, 76, 78 can be decontaminating fluid tubes for delivering decontaminating substance to the ports 60, 62, 64, 66, 68 of the of the lumen device 30. The connector ends 80, 82, 84, 86, 88 can be configured to connect the fluid passages 70, 72, 74, 76, 78 to the ports 60, 62, 64, 66, 68. The connector ends 80, 82, 84, 86, 88 can have different shapes, sizes, and constructions as suitable for a particular application and as further described below.

In the illustrated embodiment, the cradle 52 includes a cradle base 90, a cradle carriage 92, and connector supports 94, 96, and 98. The cradle base 90 can be positioned substantially stationary in the container 28. The cradle carriage 92 can be movable with respect to the cradle base 90. In the illustrated embodiment, the cradle carriage 92 may be slidably connected to the cradle base 90 in a vertical direction and the connector supports 94, 96, and 98 may be slidably connected to the cradle carriage 92 in a horizontal direction. The connector supports 94, 96, and 98 can include one or more pins 100, 102, 104, respectively, that extend substantially horizontally through diagonally extending slots 106, 108, 110, respectively, that are defined by the cradle base 90.

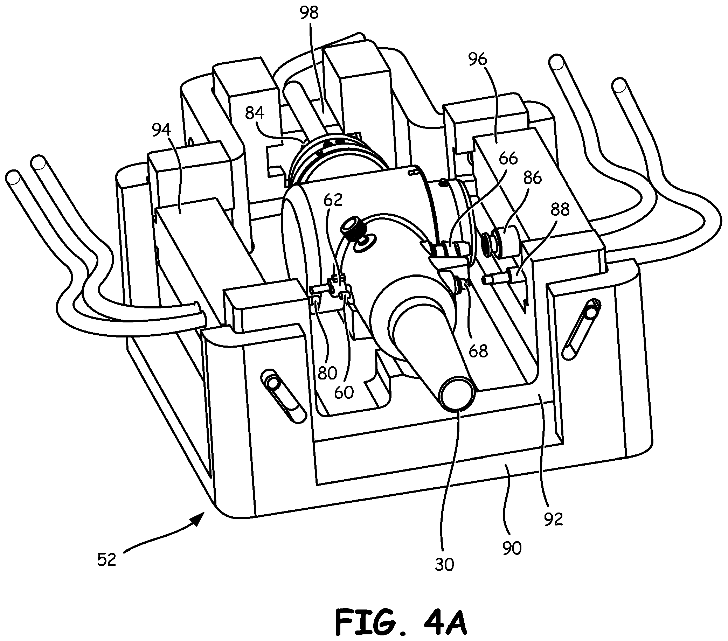

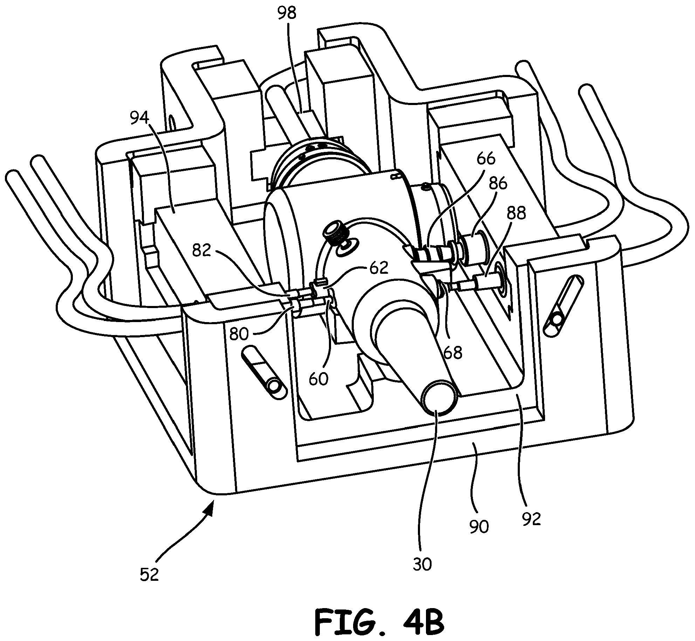

The cradle 52 can be actuated by raising and lowering the cradle carriage 92, which can raise and lower the connector supports 94, 96, and 98 and the lumen device 30 along with the cradle carriage 92. As the connector supports 94, 96, and 98 are raised and lowered, the diagonally extending slots 106, 108, 110 can cause the connector supports 94, 96, and 98 to translate laterally outward and inward. By translating the connector supports 94, 96, and 98 outward and inward, the connector ends 80, 82, 84, 86, 88 can be moved away from and moved toward the ports 60, 62, 64, 66, 68 of the lumen device 30 as further described with respect to FIGS. 4A and 4B.

In some embodiments, the cradle 52 can be actuated manually. In other embodiments, the cradle 52 can include an actuator 112 connected to the cradle 52 for actuating the cradle 52 between positions. The actuator 112 can be an electric actuator, a pneumatic actuator, a hydraulic actuator, and/or a mechanical actuator. For example, when pneumatically actuated, changes in pressure in the fluid passages 70, 72, 74, 76, 78 can cause the actuator 112 to connect and disconnect the connector ends 80, 82, 84, 86, 88 to and from the ports 60, 62, 64, 66, 68. As described herein, cradle 52 aligns or locates the lumen device 30 and connector ends 80, 82, 84, 86, 88. Cradle 52 also enables lumen device 30 and connector ends 80, 82, 84, 86, 88 to be connected in one motion.

FIG. 4A is a perspective view of the cradle 52 an open position. In the open position, the cradle carriage 92 is raised and the connector ends 80, 82 (not shown in FIG. 4A), 84, 86, 88 are spaced away from the ports 60, 62, 64 (not shown in FIG. 4A), 66, 68. Decontaminating substance flowing out of the connector ends 80, 82, 84, 86, 88 can flow over and decontaminate surfaces of the ports 60, 62, 64, 66, 68.

FIG. 4B is a perspective view of the cradle 52 in a closed position. In the closed position, the cradle carriage 92 is lowered and the connector ends 80, 82, 84 (not shown in FIG. 4B), 86, 88 are connected to the ports 60, 62, 64 (not shown in FIG. 4B), 66, 68. In one embodiment, the connector ends 80, 82, 84, 86, 88 are connected relatively tightly to the ports 60, 62, 64, 66, 68 in the closed position. In another embodiment, the connector ends 80, 82, 84, 86, 88 are connected relatively loosely to the ports 60, 62, 64, 66, 68 in the closed position.

Decontaminating substance flowing out of the connector ends 80, 82, 84, 86, 88 can flow in and through the ports 60, 62, 64, 66, 68. Thus, by actuating the cradle 52 between open and closed positions, the cradle 52 can selectively dispense decontaminating substance from the connector ends 80, 82, 84, 86, 88 to exterior surfaces of the ports 60, 62, 64, 66, 68 and through the ports 60, 62, 64, 66, 68 into lumens extending through the lumen device 30.

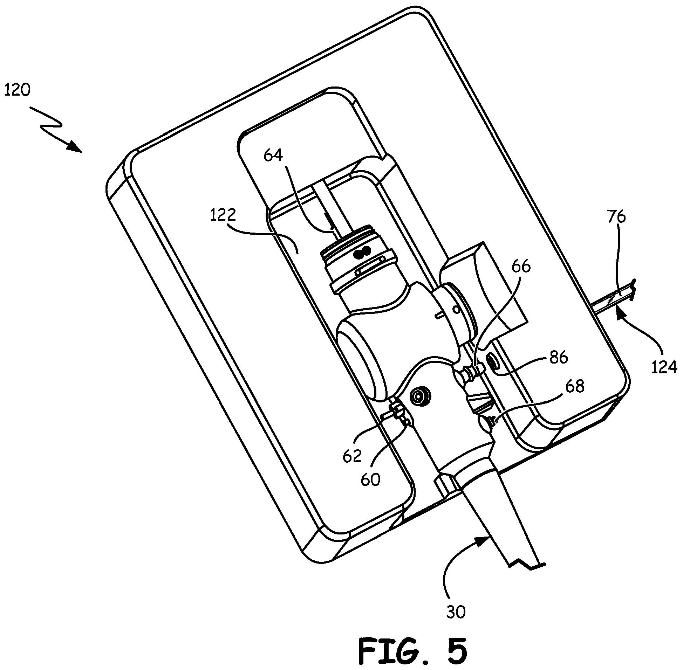

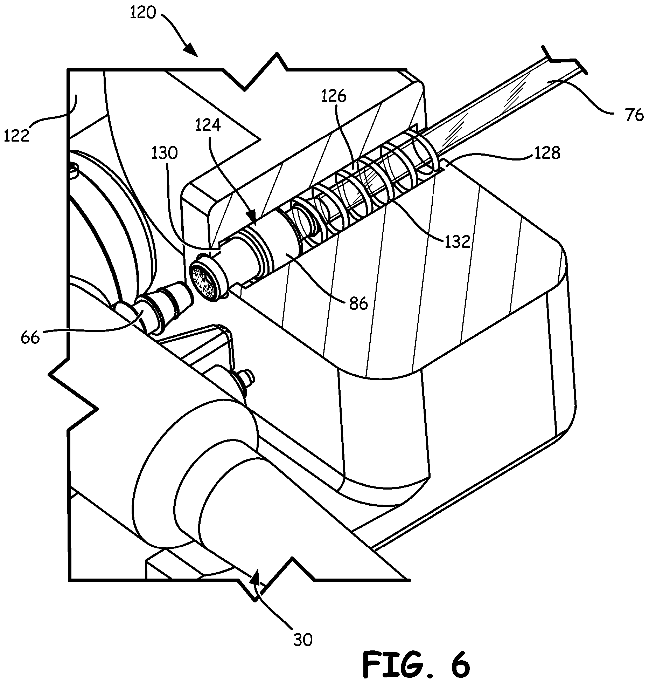

FIG. 5 is a perspective view of another embodiment of a cradle 120 for use in the container 28 (shown in FIG. 2). The cradle 120 defines a lumen device receiving area 122 for receiving the lumen device 30. The cradle 120 can include a plurality of fluid passages and connector ends for connecting to the ports 60, 62, 64, 66, 68 of the lumen device 30. In the illustrated embodiment, only the fluid passage 76 and the connector end 86 are shown for simplicity. The fluid passage 76 and the connector end 86 are part of a biased actuated connector 124 that is actuatable inward and outward for connecting to and disconnecting from the port 66. Biased actuated connector 124 is movable between a first position in which the actuated connector 124 extends towards the lumen device receiving area 122 and a second position in which the actuated connector 124 is retracted from the lumen device receiving area 122. For example, biased actuated connector 124 may be closer to the lumen device receiving area 122 in the first position as compared to the second position. As shown more clearly in FIG 6, biased actuated connector 124 includes a biasing element which biases the actuated connector 124 in the first position. In some embodiments, the biasing element biases the actuated connector 124 such that the actuated connector 124 extends towards the lumen device receiving area 122.

FIG. 6 is an enlarged, perspective, partial-sectional view of a portion of the cradle 120. The cradle 120 defines a connector guide 126 extending through the cradle 120 to the lumen device receiving area 122. The cradle 120 has rims 128 and 130 on opposite ends of the connector guide 126. The rim 128 is on an outer end of the connector guide 126 and defines a hole for the fluid passage 76 to extend at least partially through. The rim 130 is on an inner end of the connector guide 126 and defines a hole for the connector end 86 to extend at least partially through. In some embodiments, connector guide 126 can be a channel or lumen formed through the cradle 120 which enables fluid passage 76 to extend from a location outside cradle 120 to a location within cradle 120. The connector guide 126 may extend around the entire circumference of at least a portion of the fluid passage 76 or it may extend around a portion of the circumference of the fluid passage 76.

A biasing element 132 is positioned in the connector guide 126. The biasing element 132 may be any element or device which biases the actuated connector 124 to a first position. Suitable biasing elements include springs and elastomeric materials. In the illustrated embodiment, the biasing element 132 is a coil spring with a first end adjacent the rim 128 and a second end adjacent the connector end 86. The biasing element 132 is compressed as illustrated in FIG. 6, with the connector end 86 in a retracted position, spaced away from the port 66. The biasing element 132 exerts a force on the connector end 86 in an axial direction with respect to a centerline axis of the connector end 86. The biasing element 132 can bias the connector end 86 in an extended position, such that connector end 86 extends towards the port 66. In alternative embodiments, the biased actuated connector 124 and the biasing element 132 can be modified as suitable for a particular application. For example, the biasing element 132 can be configured to be in tension or can be replaced with a different type of spring such as a leaf spring.

The connector guide 126 can be sized larger than the fluid passage 76 and the connector end 86 to allow some movement within the connector guide 126. This can allow the connector end 86 and the fluid passage 76 to have some play in a radial direction, normal to an axis of the connector guide 126.

Although FIG. 6 shows only a single biased actuated connector 124, in some embodiments one or more additional biased actuating connectors 124 can be included for connecting to additional ports of the lumen device 30. Each of the biased actuating connectors 124 can be retracted individually. Alternatively, two or more of the biased actuating connectors 124 can be moved in unison. In use, the lumen device 30 can be positioned in the lumen device receiving area 122, and the biased actuating connectors 124 can then be aligned with respective ports of the lumen device 30. The biased actuating connectors 124 are released, either individually or in union, to engage with the respective ports. The decontaminating substance can be directed from the biased actuating connectors 124 to and through the respective ports.

The biased actuated connector 124 aligns the connector end 86 with the port 66, simplifying and reducing the time required to connect the fluid passage 76 to the port 66 of the lumen device 30. The biasing actuated connector 124 may also reduce the time required to disconnect the fluid passage 76 and the port 66. In some embodiments, the biasing actuated connector 124 may be controlled by a controller and the controller may command the biasing actuated connector 124 to extended and retracted positions.

As described herein, during a decontamination cycle the fluid passage 76 directs the decontamination substance into at least one lumen of the lumen device 30 for decontamination of the interior or lumen surfaces of the lumen device. In some embodiments, the surface contact between connector end 86 and port 66 prevents decontamination of the exterior surface of port 66 which is covered or masked by connector end 86. In some embodiments, biased actuated connector 124 may be retracted or disconnected from port 66 during the decontamination cycle such that the exterior of port 66 is no longer in contact with connector end 86. This exposes the exterior surface of port 66 to the decontamination substance. In some embodiments, the exterior surface of the port 66 may be contacted by decontamination substance present in the decontamination chamber. Additionally or alternatively, the decontamination substance may continue to flow through fluid passage 76 after the connector end 86 is disconnected from the port 66 and the decontamination substance exiting the fluid passage 76 may contact the exterior surface of the port 66.

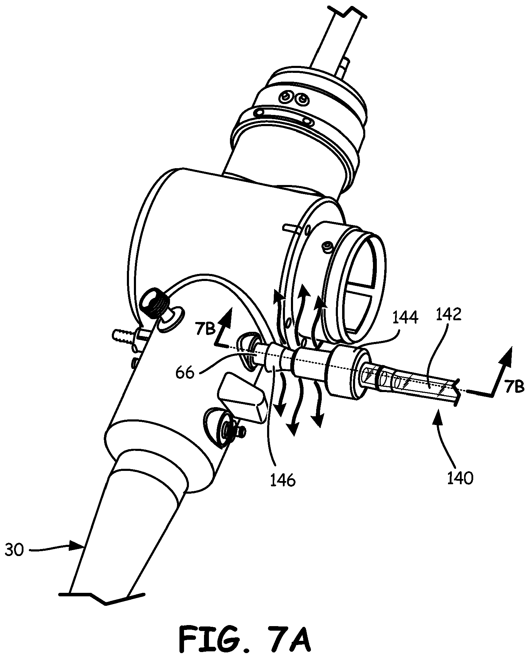

FIG. 7A is a perspective view of an embodiment of the lumen device 30 and a fluid connector 140. The fluid connector 140 includes a fluid passage 142 and a connector end 144 for connecting to the port 66 of the lumen device 30. The connector end 144 includes a connection structure 146 configured to reduce the contact surface area between the connector end 144 and the port 66. For example, the connection structure 146 may hold the connector end 144 loosely to the port 66 of the lumen device 30. In the illustrated embodiment, the connection structure 146 is a wire helix harness extending from the interior surface of connector end 144 and is configured to loosely wrap at least partially around the port 66. When the connector end 144 is positioned on the port 66, the connection structure 146 forms gaps or spaces between the interior surface of the connector end 144 and the exterior surface of the port 66 such that the connection between connector end 144 and port 66 is loose and a tight seal is not formed between the connector end 144 and the port 66. This can create what is effectively a leaking interface between the fluid connector 140 and the port 66.

By holding the connector end 144 loosely on the port 66, decontaminating substance can flow out of the connector end 144 not only into or through the port 66, but also around and over an exterior surface of the port 66. For example, less than 100% of the decontaminating substance flowing through fluid passage 140 is directed through the port 66. The decontaminating substance which does not flow through the port 66 can contact a portion of the port 66 that may otherwise be covered if the connector end 144 were to seal tightly on the port 66. In some embodiments, the connector end 144 can be sized such that the connector end 144 contacts the port 66 only intermittently while decontaminating substance flows through the fluid connector 140 and into the port 66. In other embodiments, the connector end 144 can be sized such that the connector end 144 remains spaced from the port 66 while the decontaminating substance flows through the fluid connector 140 and into the port 66. The fluid connector 140 can be used with the cradle 52 (shown in FIGS. 2, 3, 4A, and 4B), with the cradle 120 (shown in FIGS. 5 and 6), with a cradle of another design, or with a system having a design without a cradle.

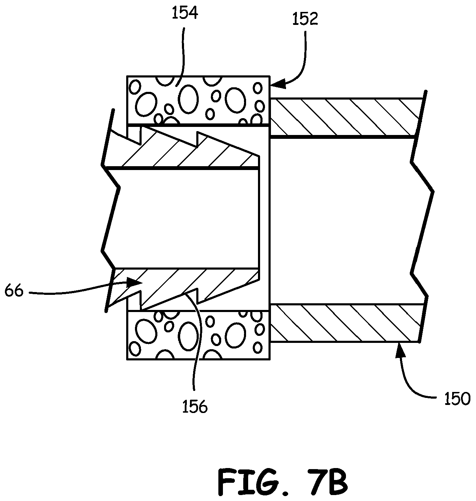

FIG. 7B is a cross-sectional view of the port 66 of the lumen device 30 (FIG. 7A) and a fluid connector 150. The fluid connector 150 is similar to the fluid connector 140 (shown in FIG. 7A) except the connection structure 146 (shown in FIG. 7A) is replaced with a connection structure 152. In the illustrated embodiment, the connection structure 152 includes a porous material 154 having a substantially annular shape. In various embodiments, the porous material 154 can be relatively rigid or relatively resilient and may for example, experience little or no deformation when connected to the port 66. In one embodiment, the porous material 154 can be a relatively hard metallic material. In another embodiment, the porous material 154 can be a relatively hard ceramic material. In a further embodiment, the porous material 154 can be a relatively hard polymeric material, such as polyolefin, including polypropylene and polyethylene, and polytetrafluoroethylene (PTFE). The connection structure 152 can hold loosely on the port 66 allowing the decontaminating substance to leak, flowing not only into the port 66 but also over an exterior surface 156 of the port 66. The fluid connector 150 can be used with the cradle 52 (shown in FIGS. 2, 3, 4A, and 4B), with the cradle 120 (shown in FIGS. 5 and 6), with a cradle of another design, or with a system having a design without a cradle.

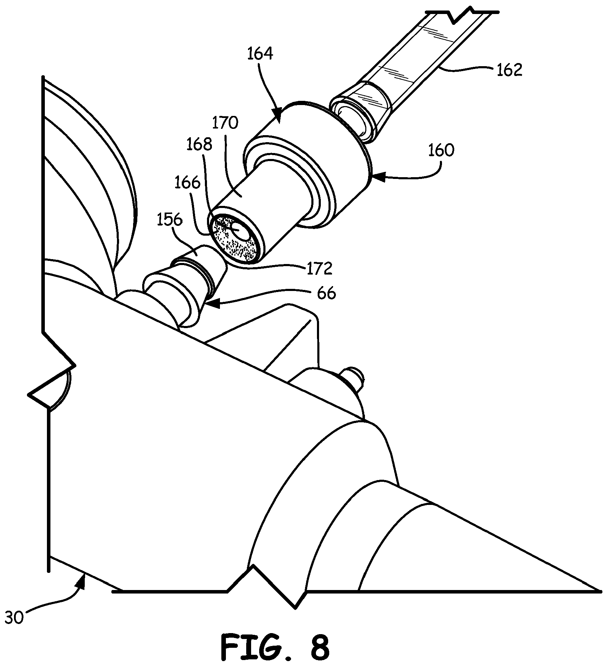

FIG. 8 is an enlarged perspective view of another embodiment of the lumen device 30 and a fluid connector 160. The fluid connector 160 includes a fluid passage 162 and a connector end 164 for connecting to the port 66 of the lumen device 30. The connector end 164 includes a textured contact surface 166 for contacting the lumen port 66.

In the illustrated embodiment, the connector end 164 is substantially tubular, with an inner surface 168 defining a fluid passage and an outer surface 170 radially outward of the inner surface 168. The textured contact surface 166 is a substantially annular surface extending between the inner surface 168 and the outer surface 170. In some embodiments, the textured contact surface 166 can be substantially concave between the inner surface 168 and the outer surface 170. In other embodiments, the textured contact surface can have a different shape suitable for the application.

When the fluid connector 160 is connected to the port 66, the textured contact surface 166 abuts a distal end 172 of the port 66. The textured contact surface 166 contacts the distal end 172 of the port 66 so as to allow fluid flow from the fluid connector 160 to flow both through and around the port 66. This can allow the decontaminating substance to effectively leak, flowing over and contacting the distal end 172 of the port 66 and the exterior surface 156 of the port 66.

In some embodiments, the textured contact surface 166 can have projections and recesses, which give the textured contact surface 166 a roughness. For example, the textured contact surface 166 can include a random roughness pattern and may be formed by media blasting the surface, such as by sand blasting or bead blasting. In other embodiments, the textured contact surface 166 can include an etched surface. In other embodiments, the textured contact surface 166 can include a knurled surface.

In some embodiments, the textured contact surface 166 can include a patterned roughness. For example, the textured contact surface 166 can include a fluted surface, having one or more ridges spirally extending along at least a portion of the surface. The fluid connector 160 can be used with the cradle 52 (shown in FIGS. 2, 3, 4A, and 4B), with the cradle 120 (shown in FIGS. 5 and 6), with a cradle of another design, or with a system having a design without a cradle. In some embodiments, the textured contact surface 166 can reduce surface contact area between the fluid connector 160 and the port 66. The textured contact surface 166 may additionally enable the leaking at the connection between the fluid connector 160 and the port 66 such that at least a portion of the exterior surface of the port 66 may be in contact with decontamination substance flowing into or through the port 66.

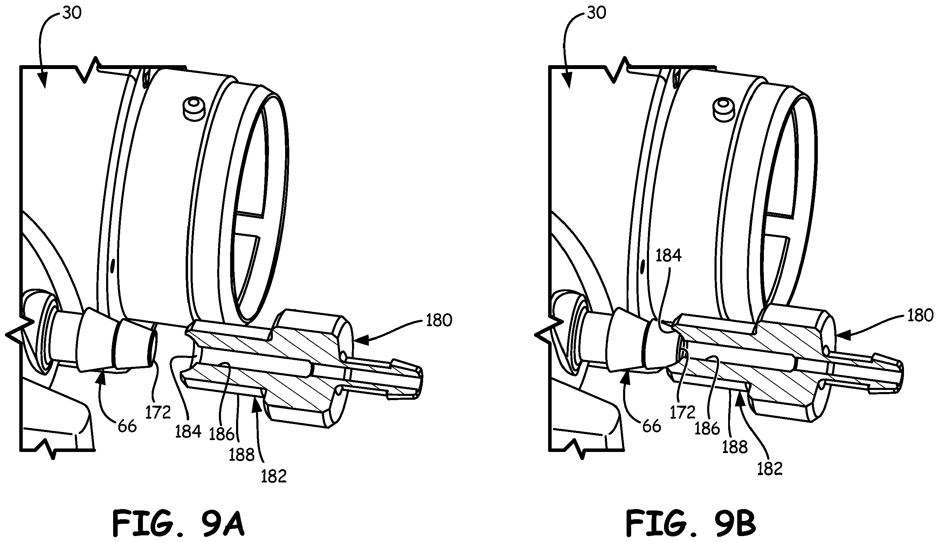

FIG. 9A is an enlarged perspective view of another embodiment of the lumen device 30 and a fluid connector 180 with the fluid connector 180 disconnected from the lumen device 30.

FIG. 9B is an enlarged perspective view of the lumen device 30 and the fluid connector 180 with the fluid connector 180 connected to the lumen device 30. The fluid connector 180 includes a connector end 182 with a contact surface 184.

In the illustrated embodiment, the connector end 182 is substantially tubular, with an inner surface 186 defining a fluid passage and an outer surface 188 radially outward of the inner surface 186. The contact surface 184 extends between the inner surface 186 and the outer surface 188. The contact surface 184 can be substantially concave between the inner surface 186 and the outer surface 188 (i.e., the contact surface 184 may curve inwards, like the inside of a bowl). In alternative embodiments, the contact surface 184 can be convex with a shape suitable for the application (i.e., the contact surface 184 may curve outwards, like the outside of a bowl).

In the illustrated embodiment, the contact surface 184 has a concave shape. If the contact surfaces of two intersecting connectors are curved, with both surfaces having different degrees of curvature, the contact area between the two connectors is reduced. If the curvature angles are different enough, the width of the contact area will be reduced to form a line. Forming the contact surface 184 into a concave shape can create a line contact between the connector end 182 and the port 66, thus reducing the locations where microbes can lie unexposed. By reducing the contact area between the contact surface 184 of the connector end 182 and the distal end 172 of the port 66, the decontaminating substance can reach both sides of the contact area and access all areas where microbes may be located. When the connector end 182 is moved from a disconnected position (shown in FIG. 9A) to a connected position (shown in FIG. 9B), the contact surface 184 can abut the distal end 172 of the port 66. If the connector end 182 is imprecisely aligned with the port 66, the shape of the contact surface 184 can cause the contact surface 184 to align with the distal end 172 of the port 66 substantially automatically. This can allow for greater control over the amount of leakage between the contact surface 184 and the distal end 172 in applications desiring relatively little leakage as well as in applications desiring more leakage.

The connector end 182 and the contact surface 184 can be sized and shaped such that a circular line contact interface is formed between the contact surface 184 and the distal end 172 of the port 66. This can reduce the amount of surface in contact during delivery of the decontaminating substance, thus increasing the amount of surface that can be contacted by the decontaminating substance. The fluid connector 180 can be used with the cradle 52 (shown in FIGS. 2, 3, 4A, and 4B), with the cradle 120 (shown in FIGS. 5 and 6), with a cradle of another design, or with a system having a design without a cradle.

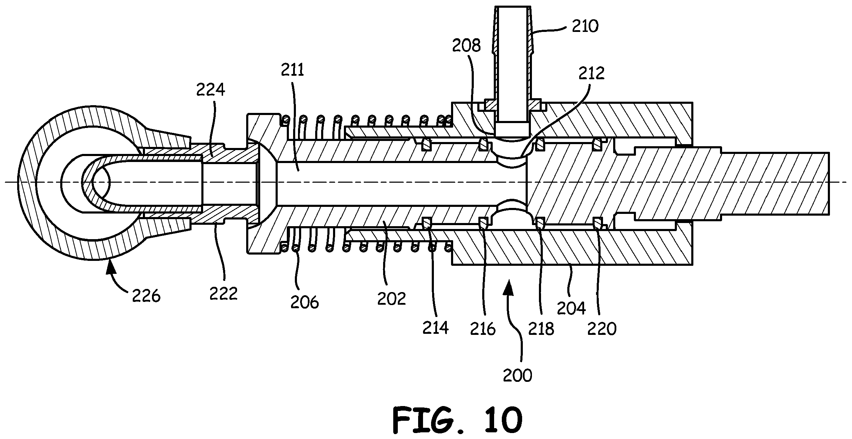

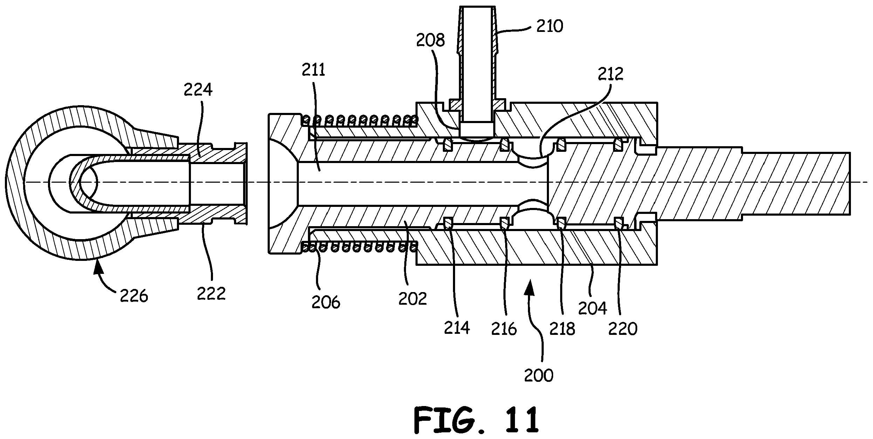

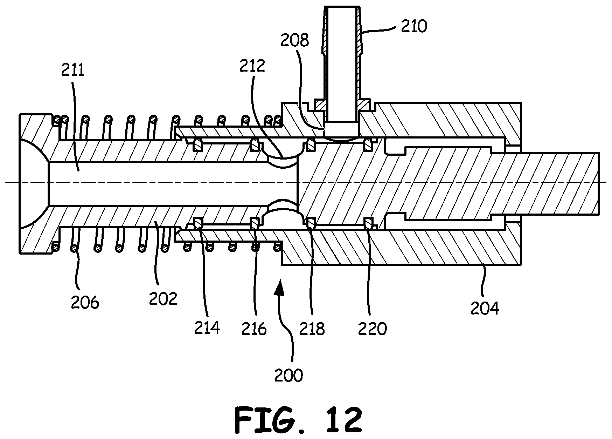

FIG. 10 is a cross-sectional view of a biased actuating connector 200 including a shuttle 202, body 204 and biasing member 206. Shuttle 202 and body 204 may be axially aligned and shuttle 202 may be slidably engaged within body 204. In some embodiments, shuttle 202 and body 204 may have substantially circular cross sectional areas and body 204 may have a larger inner diameter than the outer diameter of shuttle 202 such that shuttle 202 is received within body 204. As described herein, shuttle 202 can axially move within body 204 between a flow-through state (as shown in FIG. 10) and a shut-off state (as shown in FIGS. 11 and 12).

Biasing member 206 may be received on the body 204. Biasing member 206 biases shuttle 202 in an extended position as illustrated in FIG. 10. That is, biasing member 206 biases shuttle 202 to a position which extends away from body 204. In some embodiments, biasing member 206 can be a spring. Alternatively, biasing member 206 may be an elastomeric material with biasing properties.

Inlet port 208 may be formed in body 204. Inlet port 208 may have any suitable cross-sectional shape for receiving connector end 210. For example, inlet port 208 may have a substantially circular cross-sectional shape.

Shuttle 202 includes fluid passage 211, opening 212, and seals 214, 216, 218 and 220. Fluid passage 211 extends axially through at least a portion of shuttle 202 and seals 214 and 216 and seals 218 and 220 are positioned on either side of opening 212. In some embodiments, opening 212 may be a through-hole which extends through the diameter of shuttle 202. Seals 214, 216, 218 and 220 engage or contact the inner surface of body 204 and form a seal between shuttle 202 and body 204.

As shown in FIG. 10, the biased actuating connector 200 can fluidly connect port 222 and lumen 224 of the lumen device 226 to the connector end 210. In a first position, the opening 212 in shuttle 202 aligns with the inlet port 208 in the body 204 so that a decontaminating substance can flow from the connector end 210, through opening 212 of shuttle 202 and into lumen device 226. Seals 214, 216, 218 and 220 form seals between shuttle 202 and body 204 and prevent or reduce leakage of the decontamination substance.

FIG. 11 illustrates the biased actuating connector 200 in a retracted position, disengaged from the lumen device 226. When retracted, opening 212 does not align with inlet port 208 and the inlet port 208 is not in fluid communication with the fluid passage 211. In the retracted position, seals 214 and 216 can create a dead-head and can prevent decontamination fluid from flowing into the opening 212 and subsequently through fluid passage 211 of shuttle 202.

FIG. 12 illustrates the shuttle 202 in a fully extended position in which opening 212 does not align with inlet port 208 and the inlet port 208 is not in fluid communication with the fluid passage 211. In this position, seals 218 and 220 can create a dead-head, preventing the decontaminating substance flow into the opening 212 and subsequently through fluid passage 211 of shuttle 202. Shuttle 202 may be in a fully extended position when lumen device 226 is not present or when not properly aligned with port 222.

In some embodiments, upstream fluid flow or fluid pressure monitoring, for example by an environmental monitoring and control, may be used to determine the no-flow state, to determine that the biased actuating connector 200 is not properly engaged with the lumen device 226 (FIG. 10) or a combination thereof. For example, upstream fluid flow or fluid pressure monitoring, such as with a monitoring system, may be used to determine if shuttle 202 is in a retracted position (FIG. 11) or in an extended position (FIG. 12). Similarly, upstream fluid flow or fluid pressure monitoring may be used to determine a flow state and whether the biased actuating connector 200 is properly engaged with the lumen device 226 as shown in FIG. 10). As used herein the terms upstream and downstream are used with respect to the direction of flow of the decontaminating substance. For example, when the decontamination substance flows to the lumen device 226, shuttle 202 is upstream of the lumen device 226.

Although the decontamination substance is described as flowing from the fluid conduit through the connector to the lumen device, the decontamination fluid may flow in the opposition direction. For example, the decontamination substance may be pulled through the lumen device to the connector.

In some embodiments, a lumen device decontamination system for decontaminating a medical device having one or more lumens extending there-through includes a lumen device container including a lumen device receiving area; a fluid passage; and a biased actuated connector in fluid communication with the fluid passage and configured to fluidly connect the fluid passage to the medical device in the lumen device receiving area. The biased actuated connector is movable between a first and second position and includes a biasing element to bias the biased actuated connector in the first position. The biased actuated connector extends towards the lumen device receiving area in the first position and retracts from the lumen device receiving area in the second position. In some embodiments, the biasing element includes a spring to bias the connector in the first position. In some embodiments, the biasing element includes an elastomeric material to bias the connector in the first position.

In some embodiments, the lumen device container includes a connector guide with the biasing element slidably positioned in the connector guide. In some embodiments, the lumen device container includes first and second rims on opposite ends of the connector guide. In some embodiments, the biased actuated connector extends at least partially through holes defined by the first and second rims, and the biasing element is positioned in the connector guide between the first and second rims. In some embodiments, the biasing element exerts force on the biased actuated connector in an axial direction with respect to the biased actuated connector, and the connector guide holds the biased actuated connector so as to allow the biased actuated connector to move in one or more directions normal to the axial direction for aligning the biased actuated connector with a lumen port of the lumen device positioned in the lumen receiving area.

In some embodiments, the biased actuated connector includes a shuttle including a lumen, an opening, and a body including a port. In some embodiments, the shuttle is slidably engaged in the body, and the opening of the shuttle aligns with the port of the body. The port of the body is in fluid communication with the lumen of the shuttle when the biased actuated connector is in the first position, and the port of the body is not in fluid communication with the lumen of the shuttle when the biased actuated connector is in the second position.

In some embodiments, the biased actuated connector is movable between the first position, the second position, and third position, and when in the third position the biased actuated connector is closer to the lumen device receiving area than in the first position. In some embodiments, the port of the body is not in fluid communication with the lumen of the shuttle when the biased actuated connector is in the third position.

In some embodiments, the fluid passage has a first end connected to the biased actuated connector and a second end opposite the first end, and the decontamination system further includes a monitor system configured to monitor the pressure of the fluid passage at the second end. In some embodiments, the monitor system is configured to determine if the shuttle is in the first position or the second position.

In some embodiments, the lumen biased actuated connector includes a second fluid passage and a second biased actuated connector in fluid communication with the second fluid passage, and is configured to fluidly connect the second fluid passage to the medical device in the lumen device receiving area. In some embodiments, the second biased actuated connector is movable between a fourth and fifth position and includes a second biasing element to bias the second biased actuated connector in the fourth position, wherein the second biased actuated connector is closer to the lumen receiving area in the fourth position than in the fifth position.

In some embodiments, a method for decontaminating a medical device includes positioning a medical device in a receiving area of a lumen device container. The medical device includes a first lumen extending there-through and a first lumen port in communication with the first lumen. In some embodiments, the method includes retracting a first biased actuated connector having a first connector end, aligning a first lumen port of the medical device with the first connector end, and releasing the retracted first biased actuated connector to engage with the first lumen port.

In some embodiments, the method includes retracting a second biased actuated connector having a second connector end, aligning a second lumen port of the medical device with the second connector end, and releasing the second biased actuated connector to engage with the second lumen port.

In some embodiments, the method includes flowing a decontaminating fluid through the first biased actuated connector, to and through the first lumen port of the medical device; and flowing the decontaminating fluid through the second biased actuated connector, to and through the second lumen port of the medical device. In some embodiments, the method includes flowing a decontaminating fluid through the first biased actuated connector, to and through the first lumen port of the medical device.

In some embodiments, retracting the first biased actuated connector includes moving the biased actuated connector away from the lumen device receiving area to compress a biasing element operably connected to the first biased actuated connector. In some embodiments, the method further includes determining whether the first biased actuated connector is in the first portion with a monitoring system.

In some embodiments, the first biased actuated connector includes a shuttle slidably engaged in a body along a longitudinal axis. In some embodiments, the shuttle includes a lumen and the body includes a port, the lumen is transverse to the longitudinal axis, and the lumen is in fluid communication with the port when the first biased actuated connector is in the first position.

In some embodiments, the lumen of the shuttle is not in fluid communication with the port of the body when the first biased actuated connector is in the second position. In some embodiments, the monitoring system measures at least one of pressure and fluid flow.

In some embodiments, a decontamination system for decontaminating a medical device having one or more lumens extending there-through includes a lumen device container defining a lumen device receiving area; a retractable connector movable between first and second positions, wherein the retractable connector includes a fluid passage suitable for delivering a decontaminating fluid, and a connector end positioned at an end of the fluid passage; and an actuator operably connected to the retractable connector to actuate the retractable connector between the first and second positions. In some embodiments, the retractable connector is closer to the lumen device receiving area in the first position as compared to the second position.

In some embodiments, the lumen device container includes a cradle defining the lumen device receiving area. In some embodiments, the cradle includes a connector support connected to the retractable connector for supporting the retractable connector, and the connector support is slidably connected to the cradle.

In some embodiments, the actuator includes an electronic actuator operable to selectively move the retractable connector between the first and second positions. In some embodiments, the actuator includes a pneumatic actuator operable to selectively move the retractable connector between the first and second positions. In some embodiments, the actuator includes a mechanical actuator operable to selectively move the retractable connector between the first and second positions.

In some embodiments, the retractable connector includes a first retractable connector, wherein the actuator is a first actuator, and further includes a second retractable connector movable between third and fourth positions. In some embodiments, the retractable connector includes a second fluid passage suitable for delivering the decontaminating fluid, a second connector end positioned at a second end of the second fluid passage, and a second actuator operably connected to the second retractable connector to actuate the second retractable connector between the third and fourth positions. In some embodiments, the second retractable connector is closer to the lumen device receiving area in the third position as compared to the fourth position.

In some embodiments, a method includes positioning a medical device having a first lumen extending there-through in a receiving area of a container, aligning a first lumen port of the medical device with a first connector end of a first retractable connector in a first retracted position, and extending the first retractable connector to engage with the first lumen port, wherein the first lumen port is in fluid communication with the first lumen.

In some embodiments, the method includes aligning a second lumen port of the medical device with a second connector end of a second retractable connector in a second retracted position, and extending the second retractable connector to engage with the second lumen port, wherein the second lumen port is in fluid communication with the second lumen.

In some embodiments, the method includes flowing a decontaminating fluid through the first retractable connector to and through the first lumen port of the medical device while the first retractable connector is connected to the first lumen port, retracting the first retractable connector away from the first lumen port to form a gap between the first connector end and the first lumen port, and flowing the decontaminating fluid through the first retractable connector while the first retractable connector is retracted from the first lumen port.

In some embodiments, the first retractable connector is fluidly connected to a source of decontaminating fluid, and the first retractable connector extends and retracts in response to changes in pressure of the decontaminating fluid.

In some embodiments, a lumen device decontamination system for decontaminating a medical device having one or more lumens extending there-through includes a lumen device container including a lumen device receiving area configured to receive a medical device, and a fluid connector connected to the lumen device container and configured to deliver a decontaminating fluid to the lumen device receiving area. The fluid connector includes a fluid passage suitable for conducting flow of the decontaminating fluid; and a connector end positioned at an end of the fluid passage. In some embodiments, the connector end is configured to fluidly connect the medical device and the fluid passage, and less than 100% of the decontamination fluid flow through the fluid passage flows through the one or more lumens of the medical device.

In some embodiments, the connector end includes a protrusion extending from an inner surface configured to contact the medical device. In some embodiments, the connector end is constructed from a porous material having a substantially annular shape. In some embodiments, the porous material includes a metallic material. In some embodiments, the porous material includes a ceramic material. In some embodiments, the porous material includes a polymeric material. In some embodiments, the porous material is constructed from at least one member selected from the group consisting of polyethylene and polytetrafluoroethylene and combinations thereof.

In some embodiments, a method includes positioning a medical device having one or more lumens extending there-through in a lumen device receiving area of a lumen device container and positioning a connector end of a fluid connector proximate a lumen port of the medical device such that the connector end is configured to fluidly connect to the lumen port to allow fluid flow from the fluid conductor to flow both through the lumen port and around the lumen port. In some embodiments, less than 100% of fluid flow from the fluid conductor flows through the one or more lumens of the medical device.

In some embodiments, the method includes flowing decontaminating fluid from the fluid connector to the lumen port such that the decontaminating fluid flows both through the lumen port and around the lumen port.

In some embodiments, the connector end contacts the lumen port intermittently while the decontaminating fluid flows from the fluid connector to the lumen port. In some embodiments, the connector end remains spaced from the lumen port while the decontaminating fluid flows from the fluid connector to the lumen port. In some embodiments, the connector end is constructed from a porous material.

In some embodiments, a lumen device decontamination system for decontaminating a medical device having one or more lumens extending there-through includes a lumen device container defining a lumen device receiving area, and a fluid connector connected to the lumen device container so as to deliver a decontaminating fluid to the lumen device receiving area. The fluid connector includes a fluid passage suitable for conducting flow of the decontaminating fluid, and a connector end positioned at an end of the fluid passage, and the connector end includes a concave contact surface operable for contacting a lumen port of a medical device positioned in the lumen device receiving area.

In some embodiments, a method includes positioning a medical device having one or more lumens extending there-through in a lumen device receiving area of a lumen device container, and positioning a connector end of a fluid connector proximate a lumen port of the medical device such that a concave contact surface of the connector end contacts the lumen port of the medical device so as to allow fluid flow from the fluid conductor to flow through the lumen port.

Various modifications and additions can be made to the exemplary embodiments discussed without departing from the scope of the present invention. For example, while the embodiments described above refer to particular features, the scope of this invention also includes embodiments that do not include all of the above described features. Additionally, for example, in one embodiment, a spring loaded connector can include a connection interface that is both textured and concave with a shape of a spherical segment.

## Claims

1. A lumen device decontamination system (10) for decontaminating a medical device (30) having one or more lumens extending there-through, the lumen device decontamination system (10) comprising:
a lumen device container (28) defining a lumen device receiving area (54); and
a fluid connector (180) connected to the lumen device container (28) so as to deliver a decontaminating fluid to the lumen device receiving area (54), the fluid connector (180) comprising:
a fluid passage suitable for conducting flow of the decontaminating fluid; and
a connector end (182) positioned at an end of the fluid passage,
wherein the connector end (182) comprises a concave contact surface (184) operable for contacting a distal end (172) of a lumen port (66) of the medical device (30) positioned in the lumen device receiving area (54),
**characterised in that**
the contact surface (184) has a concavely curved shape to cause the contact surface (184) to align with the distal end (172) of the lumen port (66).

2. The decontamination system (10) of claim 1, wherein the contact surface (184) is operable for abutting the distal end (172) of the lumen port (66).

3. The decontamination system (10) of claim 1, wherein the connector end (182) is configured to conduct the decontaminating fluid from the fluid connector (180) to the lumen port (66) such that the decontaminating fluid flows both through the lumen port (66) and around the lumen port (66).

4. The decontamination system (10) of claim 1, wherein the connector end (182) is configured to contact the lumen port (66) intermittently while the decontaminating fluid flows from the fluid connector (180) to the lumen port (66).

5. The decontamination system (10) of claim 1, wherein the connector end (182) remains spaced from the lumen port (66) while the decontaminating fluid flows from the fluid connector (180) to the lumen port (66).

6. The decontamination system (10) of claim 1, wherein the connector end (182) comprises a porous material.

7. The decontamination system (10) of claim 1, wherein the connector end (182) comprises at least one of a metallic material, a ceramic material, a polymeric material, a polyethylene material, a polytetrafluoroethylene material, and combinations thereof.

8. The decontamination system (10) of claim 1, wherein the concave surface (184) comprises at least one of a textured surface, an etched surface, a media blasted surface, a knurled surface, a spiraled surface, a fluted surface, and combinations thereof.

9. The decontamination system (10) of claim 1, wherein the lumen device receiving area (54) is configured to receive the medical device (30); and
wherein the connector end (182) is configured to fluidly connect the medical device (30) and the fluid passage, and wherein less than 100% of the decontamination fluid flow through the fluid passage flows through the one or more lumens of the medical device (30).

10. The decontamination system (10) of claim 9, wherein the concave contact surface (184) is operable for abutting the distal end (172) of the lumen port (66).

11. The decontamination system (10) of claim 9, wherein the connector end (182) is configured to conduct a decontaminating fluid from the fluid connector to the lumen port (66) such that the decontaminating fluid flows both through the lumen port (66) and around the lumen port (66).

12. The decontamination system (10) of claim 9, wherein the connector end (182) is configured to contact the lumen port (66) intermittently while the decontaminating fluid flows from the fluid connector to the lumen port (66).

13. The decontamination system (10) of claim 9, wherein the connector end (182) remains spaced from the lumen port (66) while the decontaminating fluid flows from the fluid connector to the lumen port (66).

14. A method for operating a lumen device decontamination system (10) according to claim 1, comprising:
positioning a medical device (30) having one or more lumens extending there-through in the lumen device receiving area (54) of the lumen device container (28); and
positioning the connector end (182) of the fluid connector (180) proximate a lumen port (66) of the medical device (30) such that the concave contact surface (184) of the connector end (182) contacts the lumen port (66) of the medical device (30) so as to allow fluid flow from the fluid connector (180) to flow through the lumen port (66).

15. The method of claim 14, further comprising flowing a decontaminating fluid from the fluid connector (180) to the lumen port (66) such that the decontaminating fluid flows both through the lumen port (66) and around the lumen port (66).

16. The method of claim 14, further comprising positioning the connector end (182) such that the concave contact surface (184) abuts the distal end (172) of the lumen port (66).

17. The method of claim 14, further comprising flowing a decontaminating fluid from the fluid connector (180) to the lumen port (66) such that the decontaminating fluid both flows through the lumen port (66) and contacts the distal end (172) and the exterior surface of the lumen port (66).

18. The method of claim 14, wherein positioning the connector end (182) comprises positioning the connector end (182) to contact the lumen port (66) intermittently while a decontaminating fluid flows from the fluid connector (180) to the lumen port (66).

19. The method of claim 14, wherein positioning the connector end (182) comprises positioning the connector end (182) to remain spaced from the lumen port (66) while a decontaminating fluid flows from the fluid connector (180) to the lumen port (66).

## Patentansprüche

1. Dekontaminationssystem (10) für eine Vorrichtung mit Lumen zum Dekontaminieren einer medizinischen Vorrichtung (30) mit einem oder mehreren durchgehenden Lumen, wobei das Dekontaminationssystem (10) für eine Vorrichtung mit Lumen Folgendes umfasst:
einen Behälter (28) für die Vorrichtung mit Lumen, der einen Aufnahmebereich (54) für die Vorrichtung mit Lumen definiert;
und
einen Fluidverbinder (180), der mit dem Behälter (28) für die Vorrichtung mit Lumen verbunden ist, um ein dekontaminierendes Fluid an den Aufnahmebereich (54) für die Vorrichtung mit Lumen abzugeben, wobei der Fluidverbinder (180) Folgendes umfasst:
einen Fluiddurchgang, der zum Leiten des Flusses des dekontaminierenden Fluids geeignet ist; und
ein Verbinderende (182), das an einem Ende des Fluiddurchgangs positioniert ist,
wobei
das Verbinderende (182) eine konkave Kontaktfläche (184) umfasst, die zum Kontaktieren eines distalen Endes (172) eines Lumenanschlusses (66) der medizinischen Vorrichtung (30), die in dem Aufnahmebereich (54) für die Vorrichtung mit Lumen positioniert ist, betriebsfähig ist,
**dadurch gekennzeichnet, dass**
die Kontaktfläche (184) eine konkav gekrümmte Form aufweist, um die Kontaktfläche (184) zu veranlassen, sich an dem distalen Ende (172) des Lumenanschlusses (66) auszurichten.

2. Dekontaminationssystem (10) nach Anspruch 1, wobei die Kontaktfläche (184) zum Anliegen an dem distalen Ende (172) des Lumenanschlusses (66) betriebsfähig ist.

3. Dekontaminationssystem (10) nach Anspruch 1, wobei das Verbinderende (182) dazu konfiguriert ist, das Dekontaminationsfluid von dem Fluidverbinder (180) an den Lumenanschluss (66) zu leiten, sodass das Dekontaminationsfluid sowohl durch den Lumenanschluss (66) als auch um den Lumenanschluss (66) herum fließt.

4. Dekontaminationssystem (10) nach Anspruch 1, wobei das Verbinderende (182) dazu konfiguriert ist, den Lumenanschluss (66) intermittierend zu kontaktieren, während das Dekontaminationsfluid von dem Fluidverbinder (180) zu dem Lumenanschluss (66) fließt.

5. Dekontaminationssystem (10) nach Anspruch 1, wobei das Verbinderende (182) von dem Lumenanschluss (66) beabstandet bleibt, während das Dekontaminationsfluid von dem Fluidverbinder (180) zu dem Lumenanschluss (66) fließt.

6. Dekontaminationssystem (10) nach Anspruch 1, wobei das Verbinderende (182) ein poröses Material umfasst.

7. Dekontaminationssystem (10) nach Anspruch 1, wobei das Verbinderende (182) mindestens eines von einem Metallmaterial, einem Keramikmaterial, einem Polymermaterial, einem Polyethylenmaterial, einem Polytetrafluorethylenmaterial und Kombinationen davon umfasst.

8. Dekontaminationssystem (10) nach Anspruch 1, wobei die konkave Oberfläche (184) mindestens eines von einer texturierten Oberfläche, einer geätzten Oberfläche, einer mediengestrahlten Oberfläche, einer gerändelten Oberfläche, einer spiralförmigen Oberfläche, einer geriffelten Oberfläche und Kombinationen davon umfasst.

9. Dekontaminationssystem (10) nach Anspruch 1, wobei der Aufnahmebereich (54) für die Vorrichtung mit Lumen dazu konfiguriert ist, die medizinische Vorrichtung (30) aufzunehmen; und
wobei das Verbinderende (182) dazu konfiguriert ist, die medizinische Vorrichtung (30) und den Fluiddurchgang fluidisch zu verbinden, und wobei weniger als 100 % des Dekontaminationsfluidflusses durch den Fluiddurchgang durch das eine oder die mehreren Lumen der medizinischen Vorrichtung (30) fließen.

10. Dekontaminationssystem (10) nach Anspruch 9, wobei die konkave Kontaktfläche (184) zum Anliegen an dem distalen Ende (172) des Lumenanschlusses (66) betriebsfähig ist.

11. Dekontaminationssystem (10) nach Anspruch 9, wobei das Verbinderende (182) dazu konfiguriert ist, ein Dekontaminationsfluid von dem Fluidverbinder an den Lumenanschluss (66) zu leiten, sodass das Dekontaminationsfluid sowohl durch den Lumenanschluss (66) als auch um den Lumenanschluss (66) herum fließt.

12. Dekontaminationssystem (10) nach Anspruch 9, wobei das Verbinderende (182) dazu konfiguriert ist, den Lumenanschluss (66) intermittierend zu kontaktieren, während das Dekontaminationsfluid von dem Fluidverbinder zu dem Lumenanschluss (66) fließt.

13. Dekontaminationssystem (10) nach Anspruch 9, wobei das Verbinderende (182) von dem Lumenanschluss (66) beabstandet bleibt, während das Dekontaminationsfluid von dem Fluidverbinder zu dem Lumenanschluss (66) fließt.

14. Verfahren zum Betreiben eines Dekontaminationssystems (10) für eine Vorrichtung mit Lumen nach Anspruch 1, umfassend:
Positionieren einer medizinischen Vorrichtung (30) mit einem oder mehreren durchgehenden Lumen im Aufnahmebereich für die Vorrichtung mit Lumen (54) des Behälters (28) für eine Vorrichtung mit Lumen; und
Positionieren des Verbinderendes (182) des Fluidverbinders (180) in der Nähe eines Lumenanschlusses (66) der medizinischen Vorrichtung (30), sodass die konkave Kontaktfläche (184) des Verbinderendes (182) den Lumenanschluss (66) der medizinischen Vorrichtung (30) kontaktiert, um zu ermöglichen, dass ein Fluidfluss von dem Fluidverbinder (180) durch den Lumenanschluss (66) fließt.

15. Verfahren nach Anspruch 14, ferner umfassend ein Fließen eines dekontaminierenden Fluids von dem Fluidverbinder (180) zu dem Lumenanschluss (66), sodass das dekontaminierende Fluid sowohl durch den Lumenanschluss (66) als auch um den Lumenanschluss (66) herum fließt.

16. Verfahren nach Anspruch 14, ferner umfassend ein Positionieren des Verbinderendes (182), sodass die konkave Kontaktfläche (184) an dem distalen Ende (172) des Lumenanschlusses (66) anliegt.

17. Verfahren nach Anspruch 14, ferner umfassend ein Fließen eines dekontaminierenden Fluids von dem Fluidverbinder (180) zu dem Lumenanschluss (66), sodass das dekontaminierende Fluid sowohl durch den Lumenanschluss (66) fließt als auch das distale Ende (172) und die äußere Oberfläche des Lumenanschlusses (66) kontaktiert.

18. Verfahren nach Anspruch 14, wobei ein Positionieren des Verbinderendes (182) ein Positionieren des Verbinderendes (182) umfasst, um den Lumenanschluss (66) intermittierend zu kontaktieren, während ein dekontaminierendes Fluid von dem Fluidverbinder (180) zu dem Lumenanschluss (66) fließt.

19. Verfahren nach Anspruch 14, wobei ein Positionieren des Verbinderendes (182) ein Positionieren des Verbinderendes (182) umfasst, um von dem Lumenanschluss (66) beabstandet zu bleiben, während ein dekontaminierendes Fluid von dem Fluidverbinder (180) zu dem Lumenanschluss (66) fließt.

## Revendications

1. Système de décontamination de dispositif à lumière (10) pour la décontamination d'un dispositif médical (30) comprenant une ou plusieurs lumières s'étendant à travers lui, le système de décontamination de dispositif à lumière (10) comprenant :
un récipient de dispositif à lumière (28) définissant une zone de réception de dispositif à lumière (54) ; et
un raccord de fluide (180) raccordé au récipient de dispositif à lumière (28) de façon à délivrer un fluide décontaminant dans la zone de réception de dispositif à lumière (54), le raccord de fluide (180) comprenant :
un passage de fluide permettant de conduire l'écoulement du fluide décontaminant ; et
une extrémité de raccord (182) située à une extrémité du passage de fluide,
dans lequel
l'extrémité de raccord (182) comprend une surface de contact concave (184) pouvant être utilisée pour entrer en contact avec une extrémité distale (172) d'un orifice de lumière (66) du dispositif médical (30) placé dans la zone de réception du dispositif à lumière (54),
**caractérisé en ce que**
la surface de contact (184) a une forme incurvée concave permettant à la surface de contact (184) de s'aligner sur l'extrémité distale (172) de l'orifice de lumière (66).

2. Système de décontamination (10) selon la revendication 1, dans lequel la surface de contact (184) peut être utilisée pour s'appliquer sur l'extrémité distale (172) de l'orifice de lumière (66).

3. Système de décontamination (10) selon la revendication 1, dans lequel l'extrémité du raccord (182) est conçue pour conduire le fluide décontaminant du raccord de fluide (180) à l'orifice de lumière (66) de telle sorte que le fluide décontaminant s'écoule à la fois à travers l'orifice de lumière (66) et autour de l'orifice de lumière (66).

4. Système de décontamination (10) selon la revendication 1, dans lequel l'extrémité du raccord (182) est conçue pour entrer en contact avec l'orifice de lumière (66) par intermittence lorsque le fluide décontaminant s'écoule du raccord de fluide (180) à l'orifice de lumière (66).

5. Système de décontamination (10) selon la revendication 1, dans lequel l'extrémité du raccord (182) reste espacée de l'orifice de lumière (66) lorsque le fluide décontaminant s'écoule du raccord de fluide (180) à l'orifice de lumière (66).

6. Système de décontamination (10) selon la revendication 1, dans lequel l'extrémité du raccord (182) comprend un matériau poreux.

7. Système de décontamination (10) selon la revendication 1, dans lequel l'extrémité du raccord (182) comprend au moins un matériau parmi un matériau métallique, un matériau céramique, un matériau polymère, un matériau en polyéthylène, un matériau en polytétrafluoroéthylène et des combinaisons de ceux-ci.

8. Système de décontamination (10) selon la revendication 1, dans lequel la surface concave (184) comprend au moins une surface parmi une surface texturée, une surface gravée, une surface décapée par un milieu, une surface moletée, une surface spiralée, une surface cannelée et des combinaisons de celles-ci.

9. Système de décontamination (10) selon la revendication 1, dans lequel la zone de réception de dispositif à lumière (54) est conçue pour recevoir le dispositif médical (30) ; et dans lequel l'extrémité du raccord (182) est conçue pour raccorder en liaison fluidique le dispositif médical (30) et le passage de fluide, et dans lequel moins de 100 % du flux de fluide de décontamination à travers le passage de fluide s'écoule à travers une ou plusieurs lumières du dispositif médical (30).

10. Système de décontamination (10) selon la revendication 9, dans lequel la surface de contact concave (184) peut être utilisée pour s'appliquer à l'extrémité distale (172) de l'orifice de lumière (66).

11. Système de décontamination (10) selon la revendication 9, dans lequel l'extrémité du raccord (182) est conçue pour conduire un fluide décontaminant du raccord de fluide à l'orifice de lumière (66) de telle sorte que le fluide décontaminant s'écoule à la fois à travers l'orifice de lumière (66) et autour de l'orifice de lumière (66).

12. Système de décontamination (10) selon la revendication 9, dans lequel l'extrémité du raccord (182) est conçue pour entrer en contact avec l'orifice de lumière (66) par intermittence lorsque le fluide décontaminant s'écoule du raccord de fluide à l'orifice de lumière (66).

13. Système de décontamination (10) selon la revendication 9, dans lequel l'extrémité du raccord (182) reste espacée de l'orifice de lumière (66) lorsque le fluide décontaminant s'écoule du raccord de fluide à l'orifice de lumière (66).

14. Procédé pour faire fonctionner un système de décontamination de dispositif à lumière (10) selon la revendication 1, comprenant :
le positionnement d'un dispositif médical (30) comprenant une ou plusieurs lumières s'étendant à travers lui dans la zone de réception de dispositif à lumière (54) du récipient de dispositif à lumière (28) ; et
le positionnement de l'extrémité de raccord (182) du raccord de fluide (180) à proximité d'un orifice de lumière (66) du dispositif médical (30) de telle sorte que la surface de contact concave (184) de l'extrémité du raccord (182) entre contact avec l'orifice de lumière (66) du dispositif médical (30) de façon à permettre au flux de fluide venant du raccord de fluide (180) de s'écouler à travers l'orifice de lumière (66).

15. Procédé selon la revendication 14, comprenant en outre l'écoulement d'un fluide décontaminant du raccord de fluide (180) à l'orifice de lumière (66) de telle sorte que le fluide décontaminant s'écoule à la fois à travers l'orifice de lumière (66) et autour de l'orifice de lumière (66).

16. Procédé selon la revendication 14, comprenant en outre le positionnement de l'extrémité du raccord (182) de telle sorte que la surface de contact concave (184) s'applique sur l'extrémité distale (172) de l'orifice de lumière (66).

17. Procédé selon la revendication 14, comprenant en outre l'écoulement d'un fluide décontaminant du raccord de fluide (180) à l'orifice de lumière (66) de telle sorte que le fluide décontaminant s'écoule à travers l'orifice de lumière (66) et entre également en contact avec l'extrémité distale (172) et la surface extérieure de l'orifice de lumière (66).

18. Procédé selon la revendication 14, dans lequel le positionnement de l'extrémité du raccord (182) comprend le positionnement de l'extrémité du raccord (182) pour qu'elle entre en contact avec l'orifice de lumière (66) par intermittence lorsqu'un fluide décontaminant s'écoule du raccord de fluide (180) à l'orifice de lumière (66).

19. Procédé selon la revendication 14, dans lequel le positionnement de l'extrémité du raccord (182) comprend le positionnement de l'extrémité du raccord (182) pour qu'elle reste espacée de l'orifice de lumière (66) lorsque le fluide décontaminant s'écoule du raccord de fluide (180) à l'orifice de lumière (66).
